# FASCICULE DE BREVET EUROPEEN

(11) **EP 2 862 555 B1**
(45) Date de publication et mention de la délivrance du brevet: **17.05.2017**
(21) Numéro de dépôt: 14189080.6
(22) Date de dépôt: 15.10.2014
(51) Int. Cl.: A61H 15/00, A61H 15/02

(54) **Appareil de massage avec tête de massage equipee d'un rouleau a palette et d'un rouleau a surface de type lisse**
Massagegerät mit Massagekopf, der mit einer Flügelwalze und einer Walze mit glatter Oberfläche ausgestattet ist
Massage apparatus having a massage head provided with a pallet roller and a roller with a smooth surface

(30) Priorité: 17.10.2013 FR 1360119
(43) Date de publication de la demande: 22.04.2015
(73) Titulaire: SEB S.A., 69130 Ecully (FR)
(72) Inventeur: Giraud, Camille, 69001 Lyon (FR); Mandica, Franck, 69340 Francheville (FR); Paget, Monique, 38460 Optevoz (FR)
(74) Mandataire: Guéry-Jacques, Géraldine

(56) Documents cités:
- EP-A1- 0 916 330
- EP-A1- 1 932 500
- WO-A1-2010/094888
- US-A- 4 046 142
- US-B1- 6 517 499

## Description

La présente invention concerne le domaine des appareils de traitement de la peau, notamment celle du visage. L'appareil selon l'invention permet, pour le moins, le massage de la peau afin de lui donner de la tonicité. L'appareil de massage selon l'invention trouvera son application chez des personnes désireuses de soigner leur esthétique en remodelant, raffermissant et rajeunissant leur peau, notamment celle du visage.

Les appareils de massage de la peau se composent généralement d'un corps muni de moyens de motorisation et d'une tête de massage qui comprend des éléments de massage configurés pour être activés sous l'action des moyens de motorisation, par le biais d'un mécanisme de transmission. Parmi l'art antérieur existant dans ce domaine, il est connu les brevets ou demandes de brevet EP 0 666 071 A1, US 3 626 934.

Dans le document EP 0 666 071 A1, l'appareil de massage comprend deux éléments de la forme de pale qui tournent en sens inversés pour rapprocher lesdites pales qui plissent la peau. Dans une variante, cet appareil de massage comprend un élément filaire souple qui forme une boucle et dont les deux extrémités sont entraînées en rotation en sens inversés, permettant à la partie en boucle de plisser la peau.

Dans le document US 3 626 934 est décrit un appareil de massage de la peau avec deux doigts qui reproduisent un tapotement alternatif selon une direction axiale de chaque doigt vers la peau. Le système présente des doigts sous forme de marteaux de grandes surfaces et dont seulement un marteau sur deux vient en contact avec la peau.

Dans le document US4046142, l'appareil de massage du visage comporte un rouleau actionné en rotation, le rouleau est muni de palettes.

Dans le document JP2000210360, l'appareil de massage de la peau comporte un rouleau avec des picots souples ou des palettes selon plusieurs variantes de picots ou palettes pour un massage simultané et continu de battement - friction.

Dans le document EP1932500, l'appareil de massage manuel non électrique comporte un mode de réalisation à deux rouleaux, l'un à surface lisse et fixe par rapport au boitier et l'autre rotatif libre par rapport au boitier et muni de nervures protubérantes régulières.

Un document EP0916330 décrit un appareil de massage comprenant un boîtier creux délimitant une chambre d'aspiration. L'appareil comprend deux rouleaux internes logés entièrement à l'intérieur de la chambre d'aspiration et adaptés pour former un pli sur la peau et deux rouleaux externes parallèles aux rouleaux internes qui sont libres en rotation.

De tels appareils de massage permettent donc de réaliser un massage de type « étirement » ou « pincement ». Cependant ce type de massage n'est pas le seul permettant d'assurer un rajeunissement et/ou un raffermissement de la peau du visage, notamment, de sorte qu'il est apparu le besoin d'un appareil susceptible de pouvoir réaliser d'autres types de massage ou de stimulation. Ainsi, l'objet de l'invention est de proposer un appareil de massage permettant de procéder à un massage par léger pincement de manière à reproduire un massage de type « pincé Jacquet » réalisé par les professionnels de l'esthétique au moyen du pouce et de l'index de manière délicate. L'invention vise également à proposer un appareil de massage qui offre une douceur de massage de manière à pouvoir être utilisé dans des zones sensibles telles que le cou ou le visage tout en présentant des dimensions adaptées à ces zones.

Afin d'atteindre ces objectifs, l'invention concerne un appareil de massage pour le visage comprenant :
- une tête de massage qui comprend :
   - deux rouleaux de massage parallèles qui sont mobiles en rotation sur eux-mêmes selon deux axes de rotation parallèles entre eux et à la surface d'application, distants l'un de l'autre en étant séparés par une zone de travail, un premier rouleau comprenant au moins une palette qui s'étend radialement en saillie de la surface du premier rouleau, et le deuxième rouleau présentant une surface de type lisse,
   - des moyens de transmission adaptés pour entrainer les rouleaux en rotation et dans le même sens, le premier rouleau allant de l'extérieur de la zone de travail vers l'intérieur de la zone de travail et le deuxième rouleau allant de l'intérieur de la zone de travail vers l'extérieur de la zone de travail, vu depuis l'extérieur de la tête de massage,
- et un boitier d'entraînement qui porte la tête de massage et qui comprend un moteur électrique actionnant des moyens d'entrainement adaptés pour transmettre le mouvement du moteur électrique aux moyens de transmission.

Le terme « surface de type lisse » ou « surface lisse » vise une surface qui ne présente pas de palette(s) ou de protubérance(s) prononcée(s) par rapport à la dimension du diamètre du rouleau en question. Une telle surface est une surface suffisamment lisse pour rester au moins partiellement en contact avec la peau à chaque moment de la rotation du rouleau sur la peau : elle est formée de sorte que le rouleau soit en contact avec la peau sans la pincer ou la tirer. Une telle surface peut être une surface absolument lisse, une surface présentant des protubérances dont la hauteur est faible par rapport au diamètre du rouleau (moins d'un tiers du diamètre par exemple). Aussi les protubérances peu prononcées de cette surface de type lisse peuvent présenter une surface texturée, une surface cannelée, une surface avec des petites ondulations, ceci permettant de mieux accrocher la peau par contact, d'éviter un glissement sur la peau. Cette surface extérieure de type lisse sera dans la présente demande appelée ainsi car elle est lisse en comparaison avec la surface extérieure de l'autre rouleau à palette(s).

La combinaison d'un rouleau à palette avec un rouleau à surface de type lisse permet, d'une part, de reproduire le geste du pincé Jacquet et, d'autre part, d'induire un pincement efficace tout en étant indolore et inoffensif en termes de bleus par exemple. En effet, l"appareil est capable de manière surprenante de reproduire précisément le déplacement du pouce le long de la ride via le rouleau dit arrière motorisé pour « accrocher » la peau et guider l'utilisateur sans glisser sur la peau. Le pincé de l'index est reproduit grâce à un rouleau dit avant comprenant au moins une palette, motorisé et tournant à vitesse différente du rouleau arrière.

Selon une caractéristique de l'invention, les moyens de transmission sont adaptés pour entrainer les rouleaux en rotation de manière simultanée. On entend par entrainement simultané des rouleaux la rotation entrainée motorisée en même temps pour le premier et pour le deuxième rouleau. Ce terme ne limite bien entendu en rien la vitesse relative de rotation des rouleaux.

Selon une caractéristique de l'invention, les moyens de transmission sont adaptés pour assurer une vitesse de rotation du premier rouleau supérieure à la vitesse de rotation du deuxième rouleau. Ainsi, le deuxième rouleau assure la maîtrise de la vitesse d'avancement de la tête de massage sur la peau tandis que le premier rouleau reproduit le pincement.

Selon une variante de cette caractéristique, les moyens de transmission sont adaptés pour assurer une vitesse de rotation du premier rouleau entre le double et le triple de la vitesse de rotation du deuxième rouleau, bornes comprises, de préférence le double.

Selon une caractéristique de l'invention, un plan tangent aux deux rouleaux et situé vers l'extérieur de la tête de massage forme, avec un axe longitudinal du boîtier, un angle non nul et différent de l'angle droit. Une telle configuration permet d'offrir un confort d'usage en permettant de tenir le boîtier d'entraînement légèrement incliné par rapport à la surface de la peau où le massage est effectué.

Selon une autre caractéristique de l'invention, le premier rouleau comprend au moins deux palettes réparties régulièrement à sa périphérie.

Selon encore une autre caractéristique de l'invention, le premier rouleau comprend au moins une palette qui présente, en section droite transversale, une forme droite.

Selon une caractéristique de l'invention, le premier rouleau comprend au moins une palette qui présente, en section droite transversale, une forme en 8.

Selon une autre caractéristique de l'invention, le premier rouleau comprend au moins une palette qui présente, en section droite transversale, une forme allongée et effilée dont l'épaisseur se réduit en allant vers l'extrémité. Pour mieux comprendre, cette forme peut prendre la forme connue universellement d'une virgule, par exemple dont la concavité est située au repos à l'opposé du sens de rotation du premier rouleau.

Selon encore une autre caractéristique de l'invention, le premier rouleau comprend au moins une palette qui présente, en section droite transversale, une extrémité libre plus épaisse que le reste de la palette.

Selon encore une autre caractéristique de l'invention, le premier rouleau comprend au moins une palette qui présente, en section droite transversale, une forme de L majuscule dont la base est à l'extrémité libre de la palette. L'extrémité haute du L est fixée quant à elle au rouleau.

Selon une caractéristique de l'invention, la tête de massage de l'appareil de massage selon l'invention comprend une surface d'application, destinée à venir au moins partiellement en contact avec la peau. La ou les palettes du deuxième rouleau s'étendent en saillie par rapport à la surface d'appui. La surface d'application peut être agencée au moins au niveau d'un rouleau et de chaque coté dudit rouleau. Elle peut s'étendre le long de et de chaque côté des deux rouleaux et de la zone de travail. Dans un premier mode, la surface de type lisse peut être un peu en saillie par rapport à la surface d'appui ; dans un deuxième mode, elle peut être au même niveau que la surface d'application, autrement dit la surface de type lisse affleure la surface d'application. La surface d'application permet d'équilibrer l'application des deux rouleaux sur la peau en donnant un point de référence statique par rapport à la tête de massage, voire par rapport au boitier tenu par l'utilisateur.

Selon une caractéristique de l'invention, l'appareil de massage selon l'invention comprend des moyens d'application de courant électrique comprenant au moins une électrode qui est destinée à être en contact avec la peau et qui est raccordée à une unité de génération d'un courant et/ou d'une tension électriques. La mise en oeuvre de tels moyens d'application de courant électrique permet d'appliquer un faible courant sur la peau, notamment de manière à induire des phénomènes d'électrophorèse et/ou de iontophorèse qui favorisent la pénétration de principes actifs appliqués sur la peau préalablement et/ou pendant le massage.

Selon une variante de cette caractéristique, la surface d'application porte au moins une électrode.

Selon une autre variante de cette caractéristique, un rouleau porte ou forme au moins une électrode.

Selon une autre caractéristique de l'invention, l'appareil de massage comprend des moyens de diffusion de lumière. Ils sont destinés à émettre la lumière en direction du visage. La mise en oeuvre de tels moyens de diffusion de lumière permet d'effectuer un traitement de photothérapie et/ou d'activer des principes actifs appliqués sur la peau préalablement et/ou pendant le massage.

Selon une variante de cette caractéristique, les moyens de diffusion de lumière comprennent au moins une source de lumière et au moins un système optique de diffusion comprenant une face de sortie destinée à être orientée vers le visage.

Selon une autre variante de cette caractéristique, la surface d'application comprend une face de sortie de la lumière.

Selon encore une autre variante de cette caractéristique, la tête de massage comprend une face de sortie de la lumière située en regard de la zone de travail.

Selon une caractéristique de l'invention, l'appareil de massage comprend des moyens d'application de produit cosmétique. La mise en oeuvre de tels moyens d'application permet de déposer sur la peau un produit cosmétique préalablement et/ou pendant le massage.

Selon une variante de cette caractéristique, les moyens d'application de produit cosmétique comprennent au moins un capuchon qui comprend un tampon imbibé de produit cosmétique et qui est adapté de manière amovible sur la tête de massage.

Selon une autre variante de cette caractéristique, les moyens d'application de produit cosmétique comprennent un réservoir de produit cosmétique et au moins une buse de distribution raccordée à un système de prélèvement de produit comsétique dans le réservoir. Le système de prélèvement peut par exemple comprendre une pompe électrique ou encore une pompe péristaltique actionnée par les moyens d'entraînement.

Selon encore une autre variante de cette caractéristique, les moyens d'application de produit cosmétique comprennent au moins une buse de distribution située dans la tête de massage ou sur la surface d'application.

Selon une caractéristique de l'invention, la tête de massage est adaptée de manière amovible sur le boitier d'entrainement. Le caractère amovible de la tête de massage permet d'utiliser plusieurs têtes de massage interchangeables avec un même boitier.

Selon une variante de cette caractéristique, la tête de massage comprend des moyens d'identification et le boiter d'entrainement comprend des moyens de reconnaissance des moyens d'identification raccordés à une unité de commande adaptée pour contrôler le fonctionnement de l'appareil de massage en fonction de la tête de massage reconnue. La mise en oeuvre d'un tel système d'identification permet d'automatiser le réglage du fonctionnement de l'appareil de massage de sorte que l'utilisateur n'a pas à s'en préoccuper.

Bien entendu, les différentes caractéristiques, variantes et formes de réalisation de l'invention peuvent être associées les unes avec les autres selon diverses combinaisons dans la mesure où elles ne sont pas incompatibles ou exclusives les unes des autres.

Par ailleurs, diverses autres caractéristiques de l'invention ressortent de la description annexée effectuée en référence aux dessins qui illustrent des formes non limitatives de réalisation d'un appareil de massage selon l'invention.
- La figure 1 est une coupe schématique d'un appareil de massage selon l'invention.
- La figure 2 est une perspective partiellement arrachée de la tête de massage amovible équipant l'appareil de massage illustré à la figure 1.
- La figure 3 est une perspective de la tête de massage illustrée à la figure 2.
- La figure 4 est une perspective d'une autre forme de réalisation d'une tête de massage amovible pour un appareil de massage selon l'invention.
- La figure 5 est une coupe schématique de la tête de massage illustrée à la figure 4.
- La figure 6 est une perspective d'un mode alternatif des figures 1 à 5 de réalisation d'un rouleau de surface de type lisse selon l'invention illustrée.
- La figure 7 est une perspective d'un mode alternatif des figures 1 à 5 de palette du rouleau selon l'invention.

Il est à noter que sur ces figures les éléments structurels et/ou fonctionnels communs aux différentes variantes peuvent présenter les mêmes références.

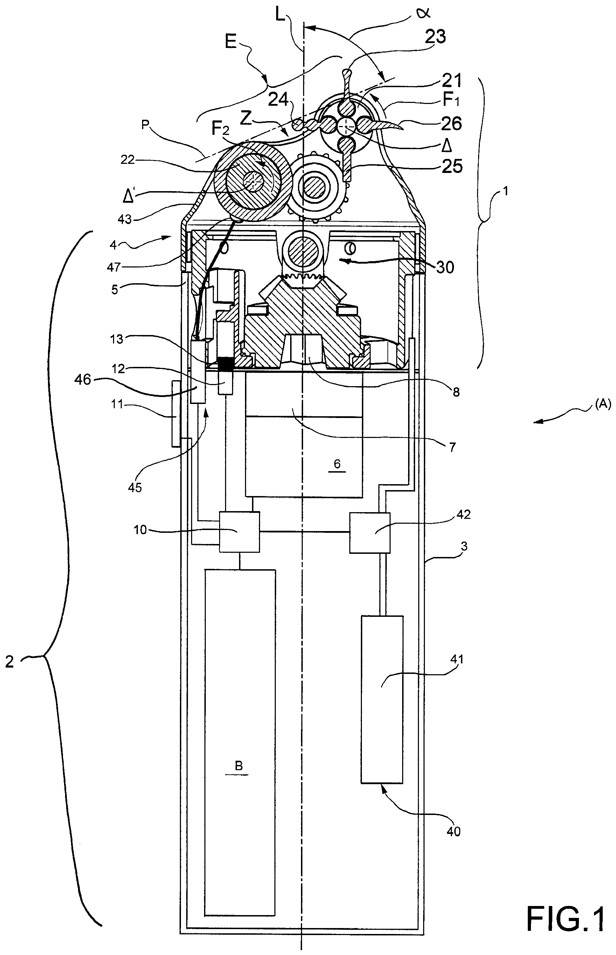

Un appareil de massage selon l'invention, tel qu'illustré à la figure 1 et désigné dans son ensemble par la référence A, comprend une tête de massage 1 adaptée de manière amovible sur un boîtier d'entraînement 2. La tête de massage 1 est conçue pour exercer une action mécanique sur la peau du visage d'un utilisateur par l'intermédiaire d'éléments de massage E entraînés par un moteur électrique.

À cet effet, le boîtier d'entraînement 2 comprend un corps allongé 3 de forme générale cylindrique qui comprend au niveau d'une de ses extrémités 4 des moyens 5 d'adaptation de manière amovible de la tête de massage 1. Les moyens d'adaptation 5 sont, selon l'exemple illustré, formés par un fourreau à l'intérieur duquel la tête de massage 1 est en partie engagée.

Le boîtier d'entraînement 2 comprend, à l'intérieur du corps 3, un moteur électrique 6 qui actionne des moyens d'entraînement 7 adaptés pour transmettre le mouvement du moteur électrique aux éléments de massage de la tête de massage 1. Selon l'exemple illustré, les moyens d'entraînement 7 comprennent un réducteur non représenté qui entraîne un arbre de sortie 8 accessible au niveau des moyens d'adaptation 5 du boîtier d'entraînement 2.

Le moteur électrique 6 est piloté par une unité de commande 10 alimentée par un bloc de batterie B disposé à l'intérieur du corps 3. Bien entendu, l'alimentation électrique de l'unité de commande 10 pourrait également être effectuée directement à partir du réseau électrique par l'intermédiaire d'un transformateur. L'unité de commande 10 est en outre raccordée à une interface de commande manuelle 11 accessible depuis l'extérieur du corps 3. L'interface de commande manuelle 11 peut par exemple comprendre un interrupteur marche-arrêt et/ou des moyens de sélection manuelle de programmes de fonctionnement.

Le boîtier d'entrainement 2 comprend aussi des moyens de reconnaissance 12 qui sont raccordés à l'unité de commande 10 et qui sont adaptés pour lire des moyens d'identification 13 portés par la tête de massage 1. L'unité de commande 10 est alors adaptée pour contrôler le fonctionnement de l'appareil de massage A en fonction de la tête de massage 1 telle que reconnue suite à la lecture des moyens d'identification 13. Le contrôle du fonctionnement de l'appareil de massage A peut notamment consister en une détermination de la vitesse de rotation du moteur électrique 6 de manière qu'elle soit adaptée au massage devant être réalisé par les éléments de massage E. Les moyens d'identification 13 peuvent, par exemple, être constitués par une puce RFID tandis que les moyens de reconnaissance 12 seront adaptés pour lire une telle puce RFID. Bien entendu, les moyens d'identification 13 et de reconnaissance 12 pourraient être réalisés de toute autre manière appropriée comme par exemple sous la forme d'un système d'identification par contact mécanique ou électrique ou encore sous la forme d'un système d'identification magnétique mettant en oeuvre des aimants permanents et des interrupteurs à lame souple (ILS) également appelés interrupteurs Reed.

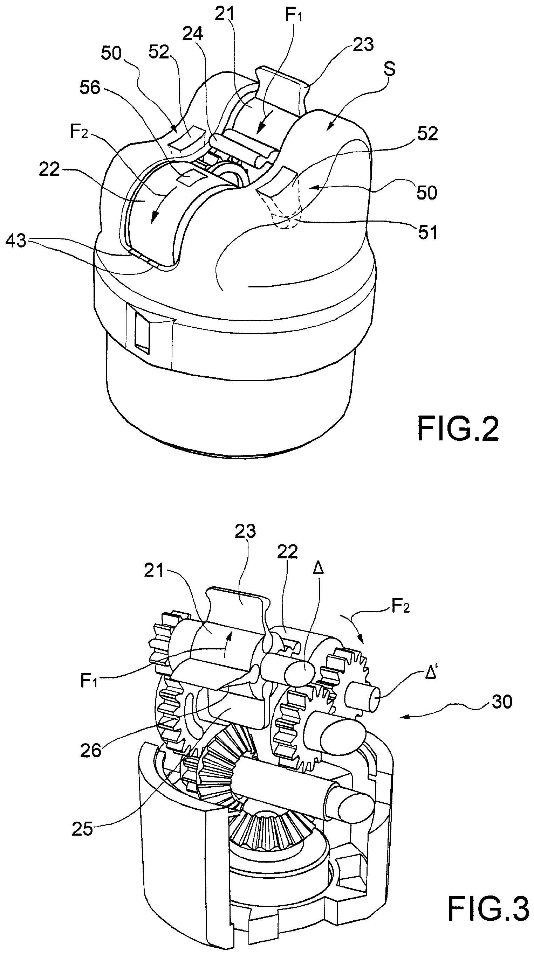

Selon l'invention, la tête de massage 1 est conçue pour réaliser un massage par pincement. À cet effet, la tête de massage 1 comprend, en tant qu'éléments de massage E, deux rouleaux de massage 21 et 22 qui sont mobiles en rotation sur eux-mêmes selon deux axes de rotation Δ et Δ' parallèles entre eux et à une surface d'application S mieux visible à la figure 2. Les deux rouleaux de massage 21 et 22 sont distants l'un de l'autre en étant séparés par une zone de travail Z. Selon l'exemple illustré, la distance entre les axes de rotation Δ et Δ' est constante.

Par ailleurs, selon l'exemple illustré, les rouleaux de massage 21 et 22 sont agencés au sein de la tête de massage 1 de manière qu'un plan P tangent aux deux rouleaux et situé vers l'extérieur de la tête de massage forme avec un axe longitudinal du boîtier d'entraînement 2 un angle α non nul et différent de l'angle droit.

Conformément à l'invention, un premier rouleau 21 comprend au moins une palette, et dans le cas présent représenté à titre d'exemple quatre palettes, 23 à 27 qui s'étendent radialement en saillie de la surface du premier rouleau 21. La surface périphérique du premier rouleau 21 est située en retrait de la surface d'application S. Les palettes 23 à 27 présentent une extension radiale suffisante pour se trouver en saillie de la surface d'application S au fur et à mesure de la rotation du premier rouleau 21. Les palettes 23 à 27 sont en outre réparties régulièrement à la périphérie du premier rouleau 21 et sont, dans le cas présent, placées à 90° les unes des autres.

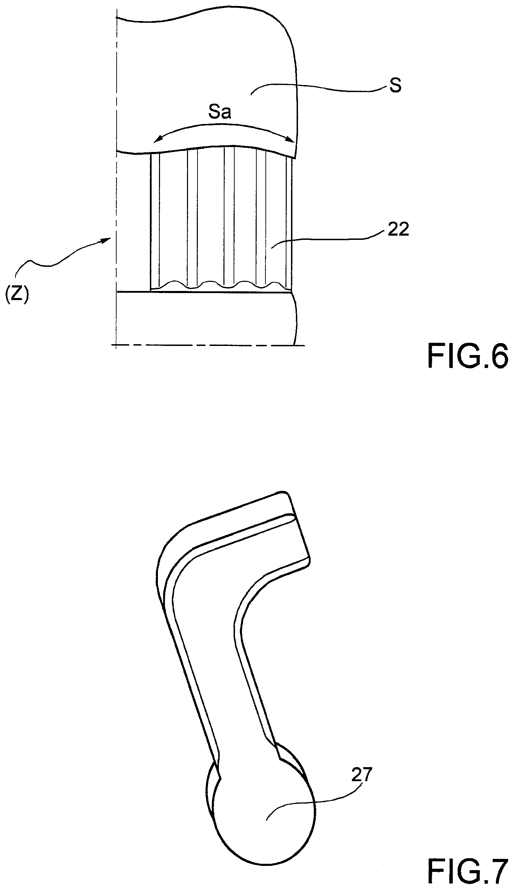

Selon l'exemple illustré, les palettes 23 à 27 possèdent des sections droites transversales, visibles en figures 1 et 7, de formes différentes et étant entendu que toutes les palettes pourraient présenter une même forme. Ainsi, la palette 23 possède, vue en section droite transversale, une extrémité libre plus épaisse que le reste de la palette 23. La palette 24 présente quant à elle, vue en section droite transversale, une forme en « 8 ». La palette 25 possède, elle, vue en section droite transversale une forme droite. Enfin, la palette 26 possède, en section droite transversale, une forme allongée et effilée dont l'épaisseur se réduit en allant vers l'extrémité. Pour mieux comprendre, cette forme peut prendre la forme connue universellement de virgule dont par exemple la concavité au repos est orientée à l'opposé du sens de rotation du premier rouleau 21 comme cela apparaîtra par la suite. Une autre forme de palette 27 est illustrée en figure 7 et présente, en section droite transversale, une forme de L majuscule dont la base est à l'extrémité libre de la palette. Chacune de ces formes permet un massage d'intensité et d'effet différents.

Le deuxième rouleau 22 présente une surface périphérique de type lisse qui s'étend en saillie de la surface d'application S ou qui s'étend à flanc de la surface d'application (S).

On a défini la surface de type lisse plus haut. Comme illustré en figures 1 à 5, la surface de type lisse du rouleau 22 peut être absolument lisse. La figure 6 illustre un mode alternatif de rouleau à surface de type lisse : la surface est cannelée parallèlement à la hauteur du rouleau, de façon régulière. Ainsi lorsque le rouleau est en contact avec la peau, il définit un angle d'appui Sa dont on peut considérer qu'au moins environ la moitié de la surface extérieure comprise dans l'angle Sa d'appui du rouleau vers la peau est en contact avec la peau.

Selon l'invention, la tête de massage 1 comprend également des moyens de transmission 30 adaptés pour entraîner les rouleaux 21 et 22 de manière simultanée dans le même sens, allant de l'extérieur de la zone de travail vers l'intérieur de la zone de travail pour le premier rouleau, et de l'intérieur vers l'extérieur de la zone de travail pour le second rouleau, vu depuis l'extérieur de la tête de massage et tel qu'indiqué par les flèches F1 et F2. Les moyens de transmission 30 sont alors adaptés pour coopérer avec les moyens d'entraînement 7 et plus particulièrement avec l'arbre de sortie 8 de manière à transmettre et transformer le mouvement de rotation du moteur électrique selon l'axe longitudinal L en des mouvements de rotation selon les axes Δ et Δ' qui présentent une direction orthogonale à celle de l'axe longitudinal L.

Selon l'exemple illustré et comme cela ressort de la figure 3, les moyens de transmission 30 comprennent un train d'engrenage comprenant, tout d'abord, deux pignons tronconiques assurant un renvoi d'angle et, ensuite, des pignons droits assurant l'entraînement ensemble, à vitesse différente des rouleaux de massage 21 et 22.

Les moyens de transmission 30 sont de préférence adaptés pour assurer une vitesse de rotation du premier rouleau 21 supérieure à celle du deuxième rouleau 22 et dans le cas présent double de celle du deuxième rouleau 22.

L'appareil de massage ainsi constitué est mis en oeuvre de la manière suivante. La surface d'application S est placée contre le visage puis l'utilisateur met en marche l'appareil de massage A au moyen de l'interface 11, les rouleaux de massage 21 et 22 s'animent alors de mouvements de rotation dans le même sens. La rotation du second rouleau 22 induit le déplacement de la tête de massage sur la peau tandis que les palettes du premier rouleau 21 reproduisent l'effet de pincement de la peau, l'espacement entre les palettes permettant de relâcher la peau successivement et au fur et à mesure de l'avancement de la tête de massage.

Le massage réalisé au moyen de l'appareil selon l'invention permet notamment de réduire les rides en stimulant la circulation sanguine dans la peau du visage ou encore dans la peau du cou. Afin d'optimiser ce traitement, l'appareil de massage A tel qu'illustré aux figures 1 à 3 comprend des moyens 40 d'application de produit cosmétique. Selon l'exemple illustré, les moyens d'application de produit cosmétique 40 comprennent un réservoir 41 situé dans le boitier d'entraînement 2 et raccordé, via une pompe de prélèvement 42, à deux buses de distribution 43 situées dans la surface d'application S et plus particulièrement à proximité immédiate du deuxième rouleau de massage 22 comme le montre la figure 2. Ainsi, le produit cosmétique est appliqué sur la peau par le deuxième rouleau de massage. La pompe de prélèvement 42 est pilotée par l'unité de commande 10 de manière à assurer la distribution de produit cosmétique lors du fonctionnement de l'appareil de massage A.

Bien entendu, la surface application S pourrait ne comprendre qu'une seule buse de distribution ou encore plusieurs buses de distribution disposées différemment. De même, la buse ou les buses de distribution de produit cosmétique pourraient être disposées à l'intérieur de la tête de massage 1 de manière à appliquer le produit cosmétique sur l'un ou l'autre voire les deux rouleaux de massage.

Par ailleurs, toujours selon l'exemple illustré aux figures 1 à 3, l'appareil de massage A comprend aussi des moyens d'application de courant électrique 45 qui comprennent une unité 46 de génération d'un courant et/ou d'une tension électrique. L'unité de génération 46 est pilotée par l'unité de commande 10. L'unité de génération 46 est raccordée, via un système de balai 47 à une électrode 56 formée par au moins partiellement la surface conductrice du deuxième rouleau 22.

Lors de l'utilisation de l'appareil de massage A, l'unité de commande 10 pilote le fonctionnement de l'unité de génération 46 de sorte par exemple qu'un microcourant de stimulation de la peau ou des muscles du visage soit appliqué ou encore qu'un phénomène d'électrophorèse favorisant l'assimilation des principes actifs du produit cosmétique soit induit.

Selon l'exemple illustré aux figures 1 à 3, la tête de massage 1 comprend des moyens 50 de diffusion de lumière en direction du visage. Dans le cas présent les moyens de diffusion 50 sont aménagés dans la tête de massage et plus particulièrement la surface d'application S. Les moyens de diffusion 50 comprennent en tant que source de lumière, deux diodes électroluminescentes 51 pilotées par l'unité de commande 10 et disposées de part et d'autre de la zone de travail Z. Ces sources de lumière 51 sont alors chacune associées à un système optique 52 formé par bloc transparent qui forme un guide de lumière et qui comprend une face de sortie de la lumière située au niveau de la surface d'appui S et donc destinée à être orientée vers le visage de l'utilisateur de l'appareil de massage A selon l'invention.

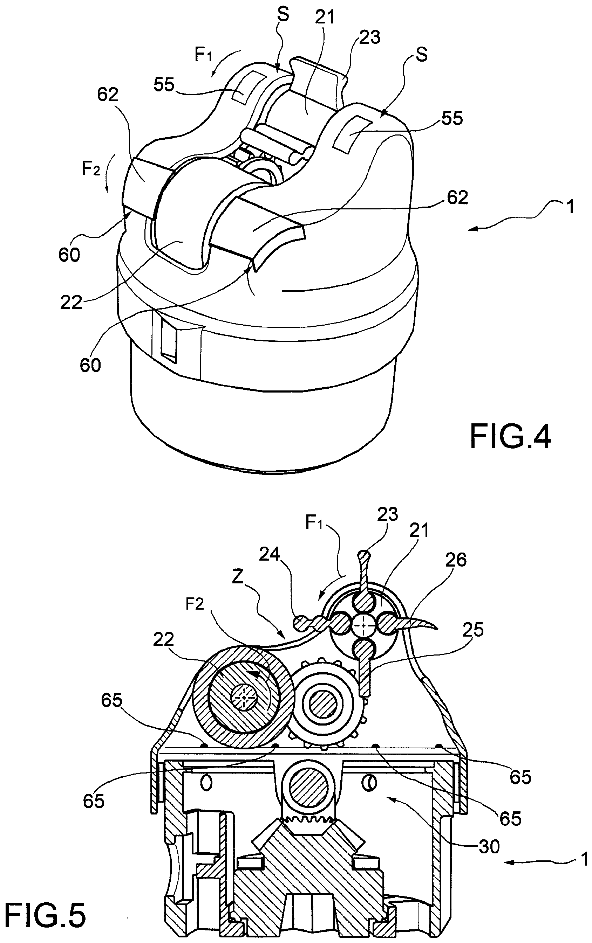

Les figures 4 et 5 illustrent une variante de réalisation d'une tête de massage pour un appareil de massage selon l'invention qui diffère de celle décrite en relation avec les figures 1 à 3, en ce que la surface d'application S comprend deux électrodes 55 raccordées à l'unité de génération 46 de courant électrique.

Toujours selon cet exemple de réalisation, la tête de massage 1 comprend en outre, en tant que moyens de distribution de produit cosmétique, deux capuchons 60 adaptés de manière amovible sur ladite tête de massage 1. Chaque capuchon 60 comprend alors un tampon 62 imbibé de produit cosmétique.

Par ailleurs selon cette variante, les moyens de diffusion 50 sont aménagés dans la tête de massage et comprennent en tant que source de lumière, des diodes électroluminescentes 65 pilotées par l'unité de commande 10. Les diodes électroluminescentes 65 éclairent l'intérieur de la tête de massage 1 de sorte que la zone de massage forme une face de sortie de la lumière. A cet égard, la partie supérieure de la tête de massage pourrait aussi être transparente de sorte que la surface d'application S formerait une face de sortie de la lumière.

Bien entendu, diverses autres modifications ou variantes de l'appareil et de la tête de massage selon l'invention, peuvent être envisagées dans le cadre des revendications annexées.

## Revendications

1. Appareil de massage pour le visage, comprenant :
- une tête de massage (1) qui comprend :
- deux rouleaux de massage (21,22) parallèles qui sont mobiles en rotation sur eux-mêmes selon deux axes de rotation parallèles entre eux et à la surface d'application (S), distants l'un de l'autre en étant séparés par une zone de travail (Z), un premier rouleau comprenant au moins une palette (23, 24, 25, 26, 27) qui s'étend radialement en saillie de la surface du premier rouleau (21), et le deuxième rouleau (22) présentant une surface de type lisse, l'appareil de massage est **caractérisé en ce qu'**il comprend en plus :
- des moyens de transmission (30) adaptés pour entrainer les rouleaux en rotation et dans le même sens, le premier rouleau allant de l'extérieur de la zone de travail (Z) vers l'intérieur de la zone de travail (Z), et le deuxième rouleau allant de l'intérieur de la zone de travail vers l'extérieur de la zone de travail, vu depuis l'extérieur de la tête de massage (1),
- et un boitier d'entraînement (2) qui porte la tête de massage (1) et qui comprend un moteur électrique (6) actionnant des moyens d'entrainement (7) adaptés pour transmettre le mouvement du moteur électrique (6) aux moyens de transmission (30).

2. Appareil de massage selon la revendication précédente, **caractérisé en ce que** les moyens de transmission (30) comprennent un train d'engrenage qui est adapté pour entrainer les rouleaux (21, 22) en rotation de manière simultanée.

3. Appareil de massage selon une des revendications précédentes, **caractérisé en ce que** les moyens de transmission (30) comprennent un train d'engrenage qui est adapté pour assurer une vitesse de rotation du premier rouleau (21) supérieure à la vitesse de rotation du deuxième rouleau (22).

4. Appareil de massage selon la revendication précédente, **caractérisé en ce que** les moyens de transmission (30) comprennent un train d'engrenage qui est adapté pour assurer une vitesse de rotation du premier rouleau (21) entre le double et le triple de la vitesse de rotation du deuxième rouleau (22), bornes comprises.

5. Appareil de massage selon l'une des revendications précédentes, **caractérisé en ce qu'**un plan (P) tangent aux deux rouleaux (21,22) et situé vers l'extérieur de la tête de massage (1) forme, avec un axe longitudinal (L) du boîtier (2), un angle (α) non nul et différent de l'angle droit.

6. Appareil de massage selon l'une des revendications précédentes, **caractérisé en ce que** le premier rouleau (21) comprend au moins deux palettes (23, 24, 25, 26, 27) réparties régulièrement à sa périphérie.

7. Appareil de massage selon l'une des revendications précédentes, **caractérisé en ce que** le premier rouleau (21) comprend au moins une palette (25, 24) qui présente, en section droite transversale, une forme droite ou une forme en « 8 ».

8. Appareil de massage selon l'une des revendications précédentes, **caractérisé en ce que** le premier rouleau (21) comprend au moins une palette (26) qui présente, en section droite transversale, une forme allongée et effilée dont l'épaisseur se réduit en allant vers l'extrémité.

9. Appareil de massage selon l'une des revendications précédentes, **caractérisé en ce que** le premier rouleau (21) comprend au moins une palette (23) qui présente, en section droite transversale, une extrémité libre plus épaisse que le reste de la palette (23).

10. Appareil de massage selon l'une des revendications précédentes, **caractérisé en ce que** le premier rouleau (21) comprend au moins une palette (27) qui présente, en section droite transversale, une forme de L majuscule dont la base est à l'extrémité libre de la palette (27).

11. Appareil de massage selon l'une des revendications précédentes, **caractérisé en ce qu'**il comprend des moyens (50) de diffusion de lumière.

12. Appareil de massage selon la revendication précédente, **caractérisé en ce que** les moyens de diffusion de lumière (50) comprennent au moins une source de lumière (51,65) et au moins un système optique de diffusion (52) comprenant une face de sortie destinée à être orientée vers le visage.

13. Appareil de massage selon la revendication précédente, **caractérisé en ce que** la surface d'application (S) comprend une face de sortie de la lumière.

14. Appareil de massage selon l'une des deux revendications précédentes, **caractérisé en ce que** la tête de massage (1) comprend une face de sortie de la lumière située en regard de la zone de travail (Z).

15. Appareil de massage selon l'une des revendications précédentes, **caractérisé en ce que** la tête de massage (1) est adaptée de manière amovible sur le boitier d'entrainement et **en ce que** la tête de massage (1) comprend des moyens d'identification (13) et **en ce que** le boiter d'entrainement comprend des moyens (12) de reconnaissance des moyens d'identification raccordés à une unité de commande (10) adaptée pour contrôler le fonctionnement de l'appareil de massage en fonction de la tête de massage (1) reconnue.

## Patentansprüche

1. Massagegerät für das Gesicht, umfassend:
- einen Massagekopf (1), der umfasst:
- zwei parallele Rollen zur Massage (21, 22), die um sich selbst gemäß zweier Drehachsen drehbeweglich sind, die zueinander und zu der Anwendungsfläche (S) parallel sind und voneinander entfernt sind, in dem sie durch einen Arbeitsbereich (Z) getrennt sind, wobei eine erste Rolle zumindest einen Flügel (23, 24, 25, 26, 27) umfasst, der sich radial von der Oberfläche der ersten Rolle (21) hervorstehend erstreckt, und wobei die zweite Rolle (22) eine Oberfläche vom glatten Typ aufweist,
wobei das Massagegerät **dadurch gekennzeichnet ist, dass** es ferner umfasst:
- Mittel zur Übertragung (30), die geeignet sind, um die Rollen in Drehung und in die gleiche Richtung anzutreiben, wobei die erste Rolle von dem Äußeren des Arbeitsbereichs (Z) in Richtung des Inneren des Arbeitsbereichs (Z) verläuft, und wobei die zweite Rolle von dem Inneren des Arbeitsbereichs in Richtung des Äußeren des Arbeitsbereichs verläuft, von dem Äußeren des Massagekopfes (1) gesehen,
- und ein Antriebsgehäuse (2), das den Massagekopf (1) trägt und das einen Elektromotor (6) umfasst, der Antriebsmittel (7) antreibt, die geeignet sind, die Bewegung des Elektromotors (6) auf die Mittel zur Übertragung (30) zu übertragen.

2. Massagegerät nach dem vorhergehenden Anspruch, **dadurch gekennzeichnet, dass** die Mittel zur Übertragung (30) einen Getriebestrang umfassen, der geeignet ist, gleichzeitig die Rollen (21, 22) in Drehung anzutreiben.

3. Massagegerät nach einem der vorhergehenden Ansprüche, **dadurch gekennzeichnet, dass** die Mittel zur Übertragung (30) einen Getriebestrang umfassen, der geeignet ist, eine Drehgeschwindigkeit der ersten Rolle (21) sicherzustellen, die höher als die Drehgeschwindigkeit der zweiten Rolle (22) ist.

4. Massagegerät nach dem vorhergehenden Anspruch, **dadurch gekennzeichnet, dass** die Mittel zur Übertragung (30) einen Getriebestrang umfassen, der geeignet ist, eine Drehgeschwindigkeit der ersten Rolle (21) zwischen dem doppelten und dreifachen der Drehgeschwindigkeit der zweiten Rolle (22) einschließlich der Grenzen sicherzustellen.

5. Massagegerät nach einem der vorhergehenden Ansprüche, **dadurch gekennzeichnet, dass** eine Ebene (P), die tangential zu den beiden Rollen (21, 22) ist und sich in Richtung zum Äußeren des Massagekopfes (1) befindet, mit einer Längsachse (L) des Gehäuses (2) einen Winkel (α) bildet, der nicht null ist und sich von dem rechten Winkel unterscheidet.

6. Massagegerät nach einem der vorhergehenden Ansprüche, **dadurch gekennzeichnet, dass** die erste Rolle (21) zumindest zwei Flügel (23, 24, 25, 26, 27) umfasst, die regelmäßig auf ihrem Umfang verteilt sind.

7. Massagegerät nach einem der vorhergehenden Ansprüche, **dadurch gekennzeichnet, dass** die erste Rolle (21) zumindest einen Flügel (25, 24) umfasst, der im Querschnitt eine gerade Form oder eine Form einer "8" aufweist.

8. Massagegerät nach einem der vorhergehenden Ansprüche, **dadurch gekennzeichnet, dass** die erste Rolle (21) zumindest einen Flügel (26) umfasst, der im Querschnitt eine längliche und sich verjüngende Form aufweist, dessen Dicke sich in Richtung des Endes verringert.

9. Massagegerät nach einem der vorhergehenden Ansprüche, **dadurch gekennzeichnet, dass** die erste Rolle (21) zumindest einen Flügel (23) umfasst, der im Querschnitt ein freies Ende aufweist, das dicker als der Rest des Flügels (23) ist.

10. Massagegerät nach einem der vorhergehenden Ansprüche, **dadurch gekennzeichnet, dass** die erste Rolle (21) zumindest einen Flügel (27) umfasst, der im Querschnitt eine Form eines großen L aufweist, dessen Basis am freien Ende des Flügels (27) ist.

11. Massagegerät nach einem der vorhergehenden Ansprüche, **dadurch gekennzeichnet, dass** es Mittel (50) zur Lichtstreuung umfasst.

12. Massagegerät nach dem vorhergehenden Anspruch, **dadurch gekennzeichnet, dass** die Mittel (50) zur Lichtstreuung zumindest eine Lichtquelle (51, 65) und zumindest ein optisches Streuungssystem (52) umfassen, das eine Austrittsfläche umfasst, die dazu bestimmt ist, zu dem Gesicht ausgerichtet zu sein.

13. Massagegerät nach dem vorhergehenden Anspruch, **dadurch gekennzeichnet, dass** die Anwendungsfläche (S) eine Austrittsfläche des Lichts umfasst.

14. Massagegerät nach einem der beiden vorhergehenden Ansprüche, **dadurch gekennzeichnet, dass** der Massagekopf (1) eine Austrittsfläche des Lichts umfasst, die sich gegenüber dem Arbeitsbereich (Z) befindet.

15. Massagegerät nach einem der vorhergehenden Ansprüche, **dadurch gekennzeichnet, dass** der Massagekopf (1) lösbar auf das Antriebsgehäuse angepasst ist, und dass der Massagekopf (1) Mittel zur Identifikation (13) umfasst, und dass das Antriebsgehäuse Mittel (12) zur Erkennung der Mittel zur Identifikation umfasst, die mit einer Steuereinheit (10) verbunden sind, die geeignet ist, den Betrieb des Massagegerätes in Abhängigkeit von dem erkannten Massagekopf (1) zu steuern.

## Claims

1. Facial massage apparatus,
comprising:
- a massage head (1) which comprises:
- two parallel massage rollers (21, 22) which are rotatable on themselves about two axes of rotation parallel with each other and with the application surface (S), set apart from each other, being separated by a working area (Z), a first roller comprising at least one paddle (23, 24, 25, 26, 27) which extends radially projecting from the surface of the first roller (21), and the second roller (22) having a smooth surface,
the massage apparatus is **characterised in that** it further comprises:
- transmission means (30) designed to rotate the rollers in the same direction, the first roller moving from outside the working area (Z) towards the inside of the working area (Z), and the second roller moving from inside the working area towards the outside of the working area, when viewed from outside the massage head (1),
- and a drive housing (2) which carries the massage head (1) and which comprises an electric motor (6) which actuates drive means (7) suitable for transmitting the drive from the electric motor (6) to the transmission means (30).

2. Massage apparatus according to the preceding claim, **characterised in that** the transmission means (30) comprise a gear train which is suitable for rotating the rollers (21, 22) simultaneously.

3. Massage apparatus according to one of the preceding claims, **characterised in that** the transmission means (30) comprise a gear train which is suitable for ensuring a greater rotational speed of the first roller (21) than the rotational speed of the second roller (22).

4. Massage apparatus according to the preceding claim, **characterised in that** the transmission means (30) comprise a gear train which is suitable for ensuring a rotational speed of the first roller (21) between double and triple the rotational speed of the second roller (22), inclusive.

5. Massage apparatus according to one of the preceding claims, **characterised in that** a tangent plane (P) to the two rollers (21, 22) which is situated towards the outside of the massage head (1) forms, with a longitudinal axis (L) of the housing (2), an angle (α) not equal to zero and different from a right angle.

6. Massage apparatus according to one of the preceding claims, **characterised in that** the first roller (21) comprises at least two paddles (23, 24, 25, 26, 27) regularly distributed on the periphery thereof.

7. Massage apparatus according to one of the preceding claims, **characterised in that** the first roller (21) comprises at least one paddle (25, 24) which has, in a transverse cross-section, a straight shape or an "8" shape.

8. Massage apparatus according to one of the preceding claims, **characterised in that** the first roller (21) comprises at least one paddle (26), which has, in a transverse cross-section, an elongated and tapered shape, the thickness whereof decreases towards the end.

9. Massage apparatus according to one of the preceding claims, **characterised in that** the first roller (21) comprises at least one paddle (23), which has, in a transverse cross-section, a thicker free end than the rest of the paddle (23).

10. Massage apparatus according to one of the preceding claims, **characterised in that** the first roller (21) comprises at least one paddle (27) which has, in a transverse cross-section, an upper case L shape, the base whereof is at the free end of the paddle (27).

11. Massage apparatus according to one of the preceding claims, **characterised in that** it comprises light diffusion means (50).

12. Massage apparatus according to the preceding claim, **characterised in that** the light diffusion means (50) comprise at least one light source (51, 65) and at least one optical diffusion system (52) comprising an output surface intended to be oriented towards the face.

13. Massage apparatus according to the preceding claim, **characterised in that** the application surface (S) comprises a light output surface.

14. Massage apparatus according to one of the two preceding claims, **characterised in that** the massage head (1) comprises a light output surface situated facing the working area (Z).

15. Massage apparatus according to one of the preceding claims, **characterised in that** the massage head (1) is removably fitted on the drive housing and **in that** the massage head (1) comprises identification means (13) and **in that** the drive housing comprises means (12) for recognising the identification means connected to a control unit (10) suitable for controlling the operation of the massage apparatus according to the massage head (1) recognised.
